# EUROPÄISCHE PATENTANMELDUNG

(11) **EP 0 583 605 A1**
(43) Veröffentlichungstag der Anmeldung: **23.02.1994**
(21) Anmeldenummer: 93111104.1
(22) Anmeldetag: 12.07.1993
(51) Int. Cl.: C09K 19/34, C09K 19/20, C09K 19/04, G11B 7/24, C09K 19/22, C09K 19/40

(54) **Definierte, oligomere flüssigkristalline Verbindungen mit smektisch flüssigkristallinen Phasen**

(30) Priorität: 22.07.1992 DE 4224083
(71) Anmelder: BASF Aktiengesellschaft, D-67063 Ludwigshafen (DE)
(72) Erfinder: Etzbach, Karl-Heinz, Dr., D-67227 Frankenthal (DE); Siemensmeyer, Karl, Dr., D-6710 Frankenthal (DE); Joachimi, Detlev, D-4059 Halle/Saale (DE); Tschierske, Carten, Dr., D-4070 Halle/Saale (DE); Agert, Olaf, D-5400 Sonderhausen (DE)

(57) **Zusammenfassung**

Oligomere flüssigkristalline Verbindungen mit smektisch flüssigkristallinen Phasen der allgemeinen Formeln I und II,

Z¹-(X¹-R¹-Y-M)ₘ I

Z²-(X²-R¹-Y-M)ₙ II,

in denen die Variablen haben folgende Bedeutung:
- Z¹: ein Alkoholat- oder Säurerest
- Z²: der n-wertige Rest einer ein- oder mehrkernigen aromatischen Verbindung
- X¹: eine chemische Bindung oder eine -CO- Gruppe;
- X²: Sauerstoff, Schwefel, -CO-O-, -O-CO-, -SO₂-, -SO₂-O, -O-SO₂-O, -NR⁴-, -CO-NR⁴-, -NR⁴-O- oder 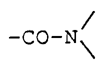 wobei R⁴ Wasserstoff oder C₁- bis C₈-Alkyl bedeuten kann,
- m: 3 bis 6,
- n: 3 bis 6;
- R¹: eine C₂- bis C₂₀-Brücke mit 2 bis 12 Brückengliedern, die durch Sauerstoff, Schwefel oder -NR⁴-unterbrochen sein können, und wobei diese Heteroeinheiten jeweils durch mindestens 2 C-Atome getrennt sind;
- Y: eine chemische Bindung, Sauerstoff, Schwefel, -CO-O-, -O-CO-, -NR⁴-, -CO-NR⁴- oder -NR⁴-CO-;
- M: eine mesogene Gruppe, die sich von einer Verbindung ableitet, welche in der flüssigkristallinen Phase eine Anisotropie der Dielektrizitätskonstanten ε mit |Δε| > 0,3 aufweist und/oder welche optisch aktiv ist.

## Beschreibung

Die vorliegende Beschreibung betrifft definierte, oligomere flüssigkristalline Verbindungen mit smektisch flüssigkristallinen Phasen der allgemeinen Formeln I und II

Z¹-(X¹-R¹-Y-M)ₘ I

Z²-(X²-R¹-Y-M)ₙ II.

Die Variablen haben folgende Bedeutung:
- Z¹: der Alkoholat- oder Säurerest Z¹/1 eines m-wertigen aliphatischen Alkohols bzw. einer m-wertigen aliphatischen Carbonsäure mit 3 bis 30 C-Atomen;
der Alkoholat- oder Säurerest Z¹/2 eines m-wertigen cycloaliphatischen Alkohols bzw. einer m-wertigen cycloaliphatischen Carbonsäure mit 5- oder 6-Ringgliedern;
für den Fall m = 3 der stickstoffhaltige Rest Z¹/3, 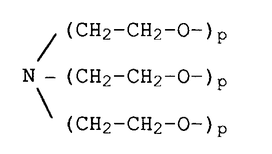 wobei p 1 oder 2 sein kann;
für den Fall M = 3 ein Rest der Strukturen Z¹/4a-d 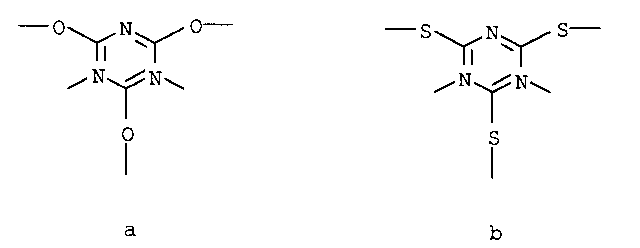 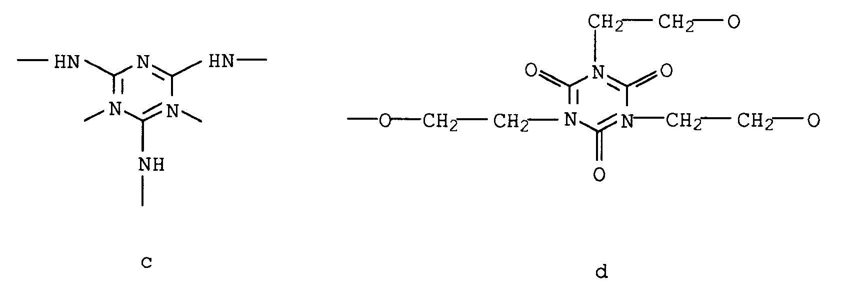 für den Fall m = 3 oder m = 4 der Säurerest Z¹/5 der Nitrilotriessigsäure bzw. der Ethylendiaminotetraessigsäure;
der Rest Z¹/6 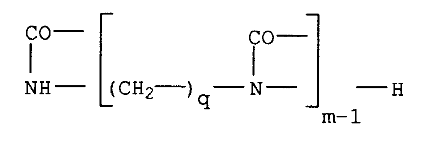 wobei q 2 oder 3 sein kann;
- Z²: der n-wertige Rest Z²/1 eines Benzols, 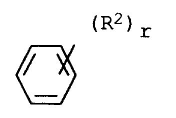 wobei R² Halogen, Cyano, Nitro, C₁- bis C₁₀-Alkyl, C₁- bis C₁₀-Alkoxy, C₁- bis C₁₀-Alkoxycarbonyl, C₁- bis C₁₀-Acyloxy oder Reste, die in vicinaler Stellung zu einem Ring verbunden sind, bedeuten kann; r für Null bis 3 steht und die Reste R² im Falle r > 1 gleich oder verschieden sein können;
der mehrkernige Rest Z²/2, 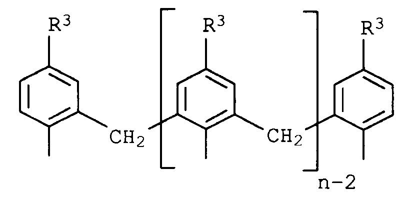 wobei R³ C₁- bis C₄-Alkyl, C₁- bis C₄-Alkoxy oder Halogen bedeuten kann;
für den Fall n= 3 der phosphorhaltige Rest Z²/3, 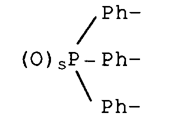 wobei s Null oder 1 sein kann;
- X¹: eine chemische Bindung oder eine -CO- Gruppe;
- X²: Sauerstoff, Schwefel, -CO-O-, -O-CO-, -SO₂-, -SO₂-O, -O-SO₂-O, -NR⁴-, -CO-NR⁴-, -NR⁴-O- oder 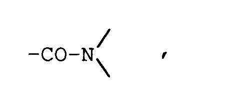 wobei R⁴ Wasserstoff oder C₁- bis C₈-Alkyl bedeuten kann, und die Maßgabe gilt, daß X² im Falle des mehrkernigen Restes Z²/2 nur -O- oder -O-CO- bedeutet;
- m: 3 bis 6, mit der Maßgabe, daß m gleich oder kleiner der Anzahl der C-Atome in Z¹ ist;
- n: 3 bis 6;
- R¹: eine C₂- bis C₂₀-Brücke mit 2 bis 12 Brückengliedern, die durch Sauerstoff, Schwefel oder -NR⁴- unterbrochen sein können, und wobei diese Heteroeinheiten jeweils durch mindestens 2 C-Atome getrennt sind;
- Y: eine chemische Bindung, Sauerstoff, Schwefel, -CO-O-, -O-CO-, -NR⁴-, -CO-NR⁴- oder -NR⁴-CO-;
- M: eine mesogene Gruppe, die sich von einer Verbindung ableitet, welche in der flüssigkristallinen Phase eine Anisotropie der Dielektrizitätskonstanten ε mit |Δε| > 0,3 aufweist und/oder welche optisch aktiv ist.

Außerdem betrifft die Erfindung Verfahren zur Herstellung der Verbindungen I und II sowie deren Verwendung in Form fester oder flüssigkristalliner optisch anisotroper Medien für die Darstellung und Speicherung von Informationen.

Flüssigkristalline Verbindungen sind optisch anisotrope Stoffe, die im Gegensatz zu Flüssigkeiten eine Fernordnung der Moleküle aufweisen, wobei sie regelmäßig ein- oder zweidimensional angeordnet sein können und flüssigkristalline Mesophasen bilden. Im Hinblick auf die räumliche Anordnung der molekularen Bausteine innerhalb des Flüssigkristalls unterscheidet man im wesentlichen 3 Phasen:
- die nematische,
- die cholesterische und
- die smektische Phase.

In nematischen Phasen sind die Einzelmoleküle eindimensional ausgerichtet. Ihr strukturelles Merkmal ist eine Parallelorientierung der Moleküllängsachsen bei statistischer Verteilung der Molekülschwerpunkte. Den Molekülen fehlt die laterale Kohäsion, so daß es nicht zu einem schichtenförmigen Aufbau wie bei den im folgenden beschriebenen smektischen Phasen kommt. Die Moleküle sind hinsichtlich ihrer langen Achsen zueinander verschiebbar, d.h. sie können frei aneinander vorbeigleiten, was ihre nidrige Viskosität bedingt. Daher sind nematische Phasen sehr viel dünnflüssiger als smektische Phasen,

Die Struktur der cholesterischen Phase, die nur mit optisch aktiven Molekülen realisierbar ist, ist eng verwandt mit der nematischen Phase. Sie wird daher auch häufig als chiral nematische Phase bezeichnet. Entsprechend der nematischen Phase sind die Moleküllängsachsen der flüssigkristallinen Verbindungen innerhalb einer quasi nematischen Schicht (Schnitt senkrecht zur Helixachse) parallel zueinander angeordnet, jedoch ändert sich die Vorzugsrichtung der Moleküllängsachsen von einer quasi nematischen Schicht zur benachbarten quasi nematischen Schicht regelmäßig um einen bestimmten Winkel. Innerhalb einer einzelnen Schicht existiert eine einheitliche Vorzugsrichtung, die gegenüber derjenigen innerhalb der benachbarten Schicht um einen konstanten Winkel gleichsinnig gedreht ist. So bildet sich über mehrere Schichten eine schraubenähnliche Anordnung der Moleküllängsachsen aus. Die Helixstruktur wird durch den chiralen Aufbau der beteiligten Moleküle hervorgerufen.

Den smektischen Phasen liegt ein zweidimensionaler Aufbau zugrunde. Durch intermolekulare Wechselwirkungen werden die langgestreckten, stäbchenförmigen, parallel ausgerichteten Molekül zu Schichten zusammengefügt und diese mit jeweils gleichen Abständen gestapelt. Hierbei können verschiedene Modifikationen auftreten, die sich in der Anordnung der Moleküle innerhalb der Schichten unterscheiden. Bekannt sind die smektischen Phasen S_{A} bis S_{I}. Beispielsweise können die Schwerpunkte der Moleküle innerhalb einer Schicht statistisch (S_{A}- und S_{C}-Phase) oder regelmäßig (z.B. S_{B}-Phase) angeordnet sein. Die Moleküllängsachsen können parallel oder geneigt zur Senkrechten auf der Schichtebene liegen. Die Moleküle können die Schichtebene nicht verlassen, da zwischen den Molekülenden kaum Wechselwirkungskräfte bestehen. Obwohl sich dadurch die Schichten noch leicht gegeneinander verschieben lassen, ist aufgrund des höheren Ordnungszustandes (zweidimensionaler Aufbau) die Viskosität smektischer Phasen höher als die nematischer Phasen.

Ferner sind smektisch flüssigkristalline Phasen bekannt, die in Abwesenheit eines äußeren elektrischen Feldes eine elektrische Spontanpolarisation aufweisen. Durch Anlegen eines äußeren elektrischen Feldes kann diese Polarisation umorientiert werden, weswegen diese Phasen als ferroelektrisch smektisch flüssigkristalline Phasen bezeichnet werden. Ein typisches Beispiel ist die chirale S_{C}-Phase (S_{C}*-Phase). Aufgrund ihrer anisotropen optischen und dielektrischen Eigenschaften lassen sie sich für elektrooptische Anzeigeelemente - kürzer Displays genannt - nutzen. So ist mit ferroelektrischen Displays auf der Basis von S_{C}*-Phasen ein extrem schnelles Einschreiben und Löschen von Zeichen möglich.

Die folgende Symbolik dient zur Kennzeichnung der flüssigkristallinen Phasen oder des flüssigkristallinen Verhaltens:
- S: symbolisiert eine smektisch flüssigkristalline Phase oder das smektisch flüssigkristalline Verhalten,
- S_{A}: symbolisiert eine smektische A Phase. In dieser sind die Moleküle in Schichten angeordnet und die Schichtnormale ist parallel zur Vorzugsrichtung der Moleküllängsachsen,
- S_{C}: symbolisiert eine smektische C Phase. In dieser sind die Moleküle in Schichten angeordnet und die Schichtnormale bildet ein Winkel mit der Vorzugsrichtung der Moleküllängsachsen, den Tiltwinkel⊖
- N: symbolisiert eine nematisch flüssigkristalline Phase oder das nematisch flüssigkristalline Verhalten,
- N*: symbolisiert eine cholesterisch (chiral nematisch) flüssigkristalline Phase oder das cholesterisch (chiral nematisch) flüssigkristalline Verhalten, wobei
- *: bedeutet, daß die flüssigkristalline Verbindung ein chirales, d.h. optisch aktives Zentrum enthält und sich daher auch optisch aktive flüssigkristalline Phasen bilden können.

Beim Schmelzen einer festen flüssigkristallinen Verbindung entsteht zunächst als flüssigkristalline Phase beispielsweise eine smektische Phase, die bei weiterer Temperaturerhöhung bei der für jede Verbindung charakteristischen Phasenübergangstemperatur entweder in eine weitere, beispielsweise nematisch flüssigkristalline Phase oder am Klärpunkt in die optisch isotrope Schmelze übergeht. Beim Abkühlen der Schmelze bilden sich bei den entsprechenden Umwandlungstemperaturen die flüssigkristallinen Phasen und schließlich der Kristall als feste Modifikation wieder aus. Diese wechselseitige Umwandlung der flüssigkristallinen Verbindung bezeichnet man als enantiotrope Umwandlung.

Flüssigkristalline Verbindungen finden sich sowohl im Bereich der niedermolekularen organischen als auch im Bereich der polymeren organischen Verbindungen. Bei den bekannten polymeren Flüssigkristallinen dienen u.a. Polyacryl- und Polymethacrylketten als polymere Hauptketten. Über polymeranaloge Umsetzungen werden Strukturelemente von niedermolekularen flüssigkristallinen Verbindungen als Seitengruppen an das Rückgrat dieser Polymeren beknüpft.

Aus der EP-B 90 282 sind flüssigkristalline Phasen bildende Polymere auf der Basis von Acrylsäure- und Methacrylsäureestern und -amiden bekannt. Die über die Ester- und Amidfunktion an die Polymerhauptkette angeknüpften Seitenketten, bestehend aus einem flexiblen, abstandhaltenden, langkettigen Molekülteil - auch Spacer genannt - und einer mesogenen Gruppe oder eines pleochroitischen Farbstoffs, nehmen Einfluß auf die Bildung von flüssigkristallinen Phasen.

Als Spacer kommen Alkylengruppen mit 2 bis 12 C-Atomen in Betracht, die linear oder verzweigt und durch Sauerstoff, Schwefel und/oder 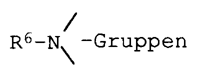
unterbrochen sein können, wobei R⁶ Wasserstoff oder gegebenenfalls ein substituiertes Alkyl ist.

Als mesogene Gruppen sind beispielsweise die in Kelker und Hatz, Handbook of Liquid Crystals, Verlag Chemie 1980, Seite 87 bis 113, genannten Gruppen möglich.

In Abhängigkeit von den Spacern und/oder den mesogenen Gruppen können die Polymeren flüssigkristalline Phasen bilden und als solche sowie in Mischungen mit niedermolekularen Flüssigkristallen in elektrooptischen Anzeigen eingesetzt werden.

Prinzipiell sind die elektrooptischen Anzeigen aus einer 1 bis 30 µm dicken flüssigkristallinen Schicht, welche sich zwischen zwei Glasplatten befindet, von denen jede auf ihrer Innenseite mit einer Elektrodenschicht bedeckt ist, und mindestens eine transparent sein muß, aufgebaut. Als transparente, elektrisch leitende Deckschichten werden vor allem mit Antimon dotierte Zinnoxidschichten und mit Zinnoxid dotierte Indiumoxidschichten verwendet.

Aus der deutschen Patentanmeldung P 39 17 196.5 gehen Monomere mit der allgemeinen Formel V hervor, 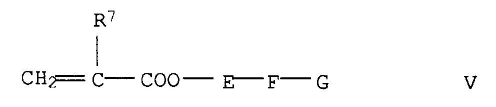
worin der Rest R⁷ ein Wasserstoff- oder Chloratom oder eine Methylgruppe, die Variable E einen flexiblen, abstandhaltenden Molekülteil, die Variable F einen aus mindestens drei linear oder angenähert linear miteinander verknüpften aromatischen Kernen aufgebauten mesogenen Molekülteil, welcher zumindest eine 2,6-Naphthylengruppe enthält, und die Variable G einen optisch aktiven chiralen Molekülteil bedeuten. Diese Monomeren finden Verwendung zur Herstellung von Polymerisaten mit chiralen mesogenen Seitengruppen, welche ferroelektrisch smektisch flüssigkristallines Verhalten zeigen.

Nachteilig bei der Verwendung von polymeren Flüssigkristallen sind die nur langsam ablaufenden Schaltvorgänge, die im oberen Millisekundenbereich bis Sekundenbereich (nematische Polymere) bzw. im unteren bis mittleren Millisekundenbereich (ferroelektrische S_{C}*-Polymere) liegen. Ein weiterer Nachteil ist, daß die polymeren Flüssigkristalle nicht wie die niedermolekularen Verbindungen mit einem einheitlichen, definierten Molekulargewicht synthetisierbar sind. Die reproduzierbare Herstellung diese Materialien stellt somit ein sehr großes synthetisches Problem dar.

Vorteilhaft für die Verwendung von polymeren Flüssigkristallen in Displayanwendungen ist jedoch die hohe mechanische Belastbarkeit der LC-Polymeren im Vergleich zu niedermolekularen Flüssigkristallen. Diese hat ihre Ursache in der wesentlich höheren Viskosität der Polymeren LC 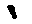 s.

Nach der Lehre von DE-A 38 27 603 sind chirale, smektische flüssigkristalline Verbindungen der allgemeinen Formel VI bekannt,

M₁*-K-M₂* VI

wobei M₁* und M₂* gleiche chirale, smektogene Gruppen sind und das Brückenglied -K- für eine bivalente Gruppe steht. Sie zeigen die Eigenschaft, daß sie gleichzeitig beim Abkühlungsprozeß aus der flüssigkristallinen Phase glasartig erstarren.

In der DE 40 11 812 wurden tetrasubstituierte Methane mit flüssigkristallinen Eigenschaften beschrieben. Diese bilden oberhalb der kristallinen Phase nematische Phasen aus. Die Materialien können wegen der geringen dielektrischen Anisotropie nicht mit dem elektrischen Feld umorientiert werden.

Der vorliegenden Erfindung lag daher die Aufgabe zugrunde, flüssigkristalline Verbindungen zu finden, die eine hohe dielektrische Anisotropie aufweisen, eine hohe Fließviskosität bei niedriger Rotationsviskosität zeigen und damit eine hohe mechanische Stabilität aufweisen sowie kampatibel mit gängigen flüssigkristallinen Mischungen zu sein.

Demgemäß wurden die eingangs definierten flüssigkristallinen Verbindungen I und II gefunden.

Ferner wurden verschiedene, im folgenden näher beschriebene Verfahren für die Herstellung der flüssigkristallinen Verbindungen I und II gefunden sowie deren Verwendung allein, in Mischungen untereinander und mit anderen flüssigkristallinen und/oder nicht flüssigkristallinen Verbindungen in Form fester oder flüssigkristalliner optisch anisotroper Medien für die Darstellung und Speicherung von Informationen.

Den durch die allgemeinen Formeln I und II

Z¹-(X¹-R¹-Y-M)ₘ I

Z²-(X²-R¹-Y-M)ₙ II

beschriebenen flüssigkristallinen Verbindungen liegen zentrale Molekülteile - auch Zentraleinheiten genannt - zugrunde, die durch die Variablen Z¹ und Z² symbolisiert werden. Die Variable X¹ bezeichnet eine chemische Bindung oder eine -CO-Gruppe, die Variable X² steht ganz allgemein für Heteroeinheiten, die wie X¹ an eine C₂- bis C₂₀-Brücke gebunden ist, welche durch die Variable Y steht wiederum für eine chemische Bindung oder für Heteroeinheiten und die Variable M für mesogene Gruppen, die sich von Verbindungen ableiten, welche in der flüssigkristallinen Phase eine Anisotropie der Dielektrizitätskonstanten ε mit |Δε| > 0,3 aufweisen und/oder welche optisch aktiv sind.

Die Variable m bedeutet eine Zahl von 3 bis 6, mit der Maßgabe, daß m gleich oder kleiner der Anzahl der C-Atome in Z¹ ist, die Variable n bedeutet eine Zahl von 3 bis 6.

Die Zentraleinheit Z¹ steht für Reste, die sich beispielsweise von folgenden Verbindungen ableiten:
von aliphatischen Alkoholen wie
Glycerin,
1,2,4-Butantriol,
2-Methyl-2-hydroxymethyl-1,3-propandiol,
2-Ethyl-2-hydroxymethyl-1,3-propandiol,
1,2,3,4-Butantetraol,
Pentaerythrit,
Xylit,
Mannit und
Sorbit,
von aliphatischen Carbonsäuren wie
1,2,3-Propantricarbonsäure,
1,1,4-Butantricarbonsäure,
1,2,3,4-Butantetracarbonsäure,
Zitronensäure und
2-Hydroxy-nonadecyl-1,2,3-tricarbonsäure,
von cycloaliphatischen Alkohllen mit 5- oder 6-Ringgliedern wie
1,2,3,4-Tetrahydroxy-cyclopentan,
1,2,3-Trihydroxy-cyclohexan,
1,2,4-Trihydroxy-cyclohexan,
1,3,5-Trihydroxy-cyclohexan,
1,2,3,4-Tetrahydroxy-cyclohexan,
1,2,3,5-Tetrahydroxy-cyclohexan,
1,2,4,5-Tetrahydroxy-cyclohexan,
1,2,3,4,5-Pentahydroxy-cyclohexan und
1,2,3,4,5,6-Hexahydroxy-cyclohexan,
von cycloaliphatischen Carbonsäuren mit 5- oder 6-Ringgliedern wie
1,2,3-Cyclopentantricarbonsäure,
1,2,4-Cyclopentantricarbonsäure,
2-Methyl-1,2,3-cyclopentantricarbonsäure,
3-Methyl-1,2,4-cyclopentantricarbonsäure,
1,1,2,2-Cyclopentantetracarbonsäure,
1,2,2,4-Cyclopentantetracarbonsäure,
1,1,3,3-Cyclopentantetracarbonsäure,
1,2,3,4-Cyclopentantetracarbonsäure,
1,2,3,4,5-Cyclopentanpentacarbonsäure,
1,1,4-Cyclohexantricarbonsäure,
1,2,4-Cyclohexantricarbonsäure,
1,3,5-Cyclohexantricarbonsäure,
1,1,3,3-Cyclohexantetracarbonsäure,
1,1,4,4-Cyclohexantetracarbonsäure,
1,2,3,4-Cyclohexantetracarbonsäure,
1,2,4,5-Cyclohexantetracarbonsäure,
1,1,3,3,5-Cyclohexanpentacarbonsäure und
1,2,3,4,5,6-Cyclohexanhexacarbonsäure,
von Alkoholaminen wie
Triethanolamin,
Triisopropanolamin und
Aminoethylethanolamin,
von Triazinderivaten wie
Cyanursäure,
Thiocyanursäure,
Melamin und
Trishydroxyethylisocyanurat,
von Nitrilotriessigsäure und Ethylendiaminotetraessigsäure,
und von dem Rest,

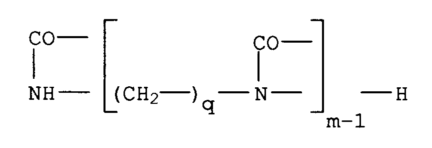

wobei q 2 oder 3 sein kann.

Besonders gut geeignete Zentraleinheiten Z¹ sind Glycerin, Pentaerythrit, Mannit und Zitronensäure.

Die Zentraleinheit Z² steht für n-wertige Reste Z²/1 eines Benzols, 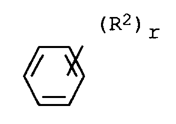
wobei R² Halogen, Cyano, Nitro, C₁- bis C₁₀-Alkyl, C₁- bis C₁₀-Alkoxy, C₁- bis C₁₀-Alkoxycarbonylk, C₁- bis C₁₀-Acyloxy oder Reste, die in vicinaler Stellung zu einem Ring verbunden sind, bedeuten kann, r für Null bis 3 steht und die Reste R² im Falle r > 1 gleich oder verschieden sein können.

Beispielsweise steht die Zentraleinheit Z² für Reste Z²/1, die sich von folgenden Verbindungen wie
1,2,3-Trihydroxybenzol,
1,2,4-Trihydroxybenzol,
1,3,5-Trihydroxybenzol,
1,2,3,4-Tetrahydroxybenzol,
1,2,3,5-Tetrahydroxybenzol,
1,2,4,5-Tetrahydroxybenzol,
Hexahydroxybenzol,
1,2,3-Benzoltricarbonsäure,
1,2,4-Benzoltricarbonsäure,
1,3,5-Benzoltricarbonsäure,
3,4,5-Trihydroxybenzoesäure,
3,4,5-Trihydroxybenzoesäuremethylester,
1,2,3-Trihydroxytoluol,
2,4,5-Trihydroxytoluol,
2,4,6-Trihydroxytoluol,
3,4,5-Trihydroxytoluol,
Pyromellithsäureanhydrid,
1,2,3,4-Tetrahydroxynaphthalin,
2,3,4-Trihydroxyanthracen,
1,2,3-Trihydroxy-9,10-anthrachinon und
1,2,4-Trihydroxy-9,10-anthrachinon
ableiten.

Ferner steht die Variable Z² für den mehrkernigen Rest Z²/2 mit der Formel 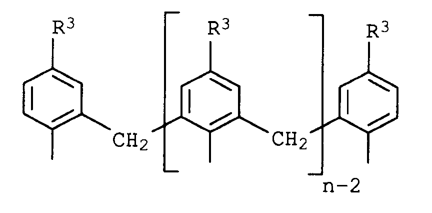
wobei R³ C₁- bis C₄-Alkyl, C₁- bis C₄-Alkoxy oder Halogen bedeuten kann.

Für den Fall n = 3 kann die Zentraleinheit Z²/3 für den phosphorhaltigen Rest stehen, 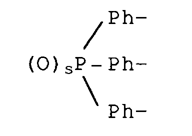
wobei s Null oder 1 sein kann.
Bevorzugte Verbindungen sind
1,3,5-Trihydroxybenzol,
Pyromellithsäureanhyrid und
3,4,5-Trihydroxybenzoesäuremethylester sowie
der Rest 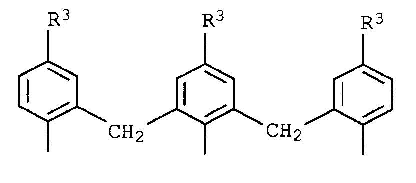
Bei den Variablen X² handelt es sich im wesentlichen um die Heteroeinheiten
Sauerstoff, Schwefel, -CO-O-, -O-CO-, -SO₂-, -SO₂-O, -O-SO₂-O, -NR⁴-, -CO-NR⁴-, -NR⁴-O- oder 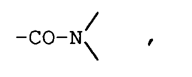
wobei R⁴ Wasserstoff oder C₁- bis C₈-Alkyl bedeuten kann, und die Maßgabe gilt, daß X² im Falle des mehrkernigen Restes Z²/2 nur -O-oder -O-CO- bedeutet.

Bevorzugt sind Sauerstoff, -CO-O- und -O-CO-.

Die Variable R¹ steht für eine C₂- bis C₂₀-Brücke mit 2 bis 12 Brückengliedern, die durch Sauerstoff, Schwefel oder -NR⁴- unterbrochen sein können, und wobei diese Hteroeinheiten jeweils durch mindestens 2 C-Atome getrennt sind.

Gut geeignete Brückenglieder R¹ sind beispielsweise
-(CH₂)₂-, -(CH₂)₃-, -(CH₂)₄-, -(CH₂)₅-, -(CH₂)₆-, -(CH₂)₇-, -(CH₂)₈-, -(CH₂)₁₀-, -(CH₂)₁₂, -(CH₂-O-CH₂)₂-, -(CH₂)₂-S-(CH₂)₂-,

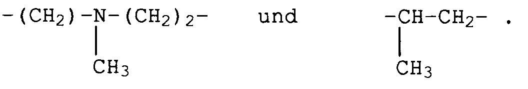

Besonders gut geeignete Brückenglieder R¹ sind -(CH₂)₅-, -(CH₂)₆-, -(CH₂)₇- und -(CH₂)₁₀-.

Die Variable Y kennzeichnet eine chemische Bindung, Sauerstoff, Schwefel, -CO-O-, -O-CO-, -NR⁴-, -CO-NR⁴- oder -NR⁴-CO-, wobei R⁴ Wasserstoff oder C₁- bis C₈-Alkyl bedeuten kann.

Besonders bevorzugt ist Sauerstoff.

Gut geeignete mesogene Gruppen M sind Gruppen der allgemeinen Formel III,

-A-(B-A-)-Bₜ-(D¹)ᵤ III

in der die Variable A eine 1,4-Phenylen- oder eine 1,3,4-Thiadiazol- oder 1,3,4-Oxdiazol- oder eine 1,4-Cyclohexylengruppe bedeuten. In der 1,4-Phenylen- und 1,4-Cyclohexylengruppen können bis zu zwei der folgenden Heteroatome Sauerstoff, Schwefel oder Stickstoff enthalten sein mit der Maßgabe, daß sie für die Heteroatome Sauerstoff und Schwefel nicht benachbart sein dürfen.

Des weiteren können die 1,4-Phenyl- und 1,4-Cyclohexylen-Ringe bis zu zweimal mit Cl, Be, F, CN oder NO₂-Gruppen substituiert sein,

die Variable B eine chemische Bindung oder eine der folgenden Brückenglieder
-CO-O-, -O-CO-, -CH₂-CH₂-, -CH₂-O-, -O-CH₂-, -CH₂-S-, -S-CH₂-, -C≡C-, -CH=CH-, -CH=N-, -N=CH-, -N=N-, 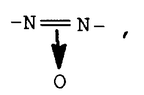
-CH=CH-CO-O- oder -O-CO-CH=CH-,
die Variable D¹ eine der folgenden Substituenten -O-CO-R⁵ oder -CO-O-R⁵, wobei R⁵ ein linearer C₁- bis C₂₀-Alkylrest, der durch Sauerstoff unterbrochen sein kann, und der durch Fluor, Chlor, Brom, Cyano oder Methyl asymmetrisch substituiert sein kann, bedeutet,
die Variable t eine Zahl von 1 bis 4, mit der Maßgabe, daß die Gruppen A und B verschieden sein können, und
die Variable u 1, wenn D¹ mit einem aromatischen Ring verknüpft, und 1 oder 2, wenn D¹ mit einem Cyclohexylenring verknüpft ist,
bedeuten.

Bevorzugte mesogene Gruppen M sind: 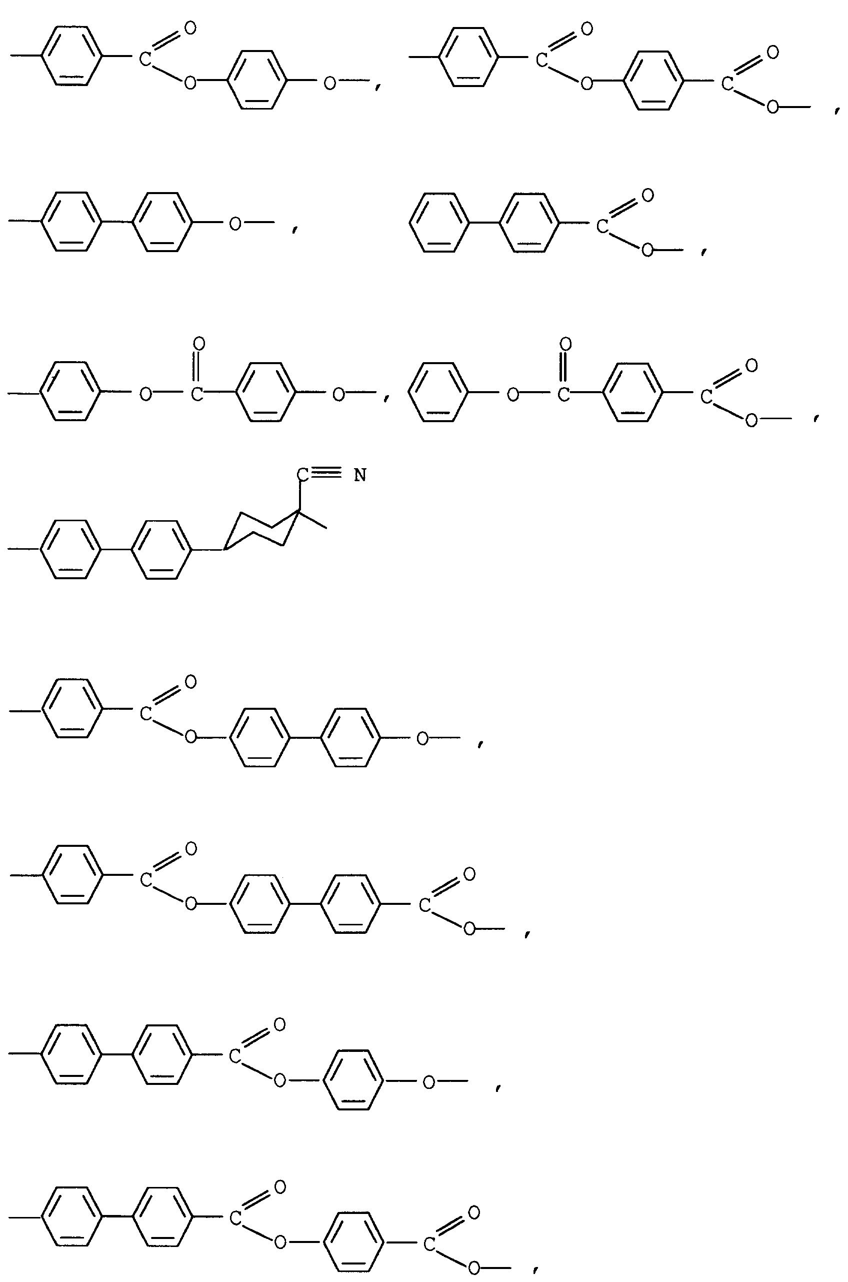 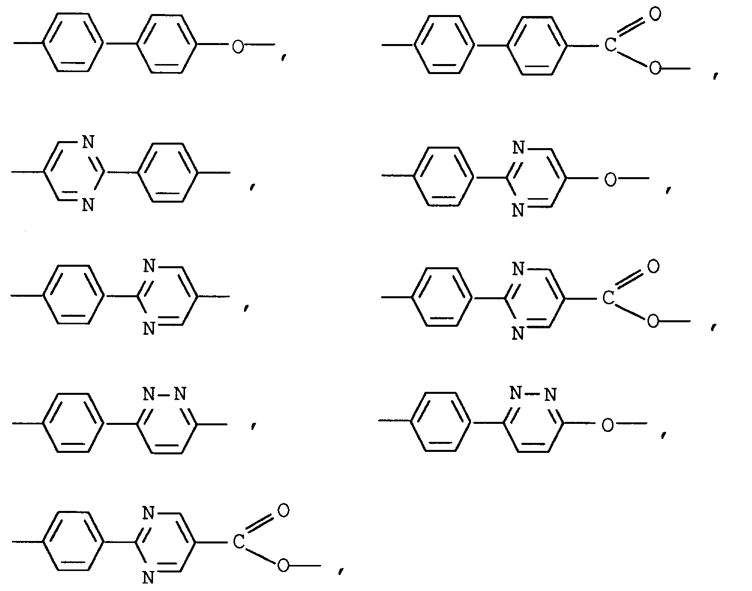
Weitere gut geeignete mesogene Gruppen M sind Gruppen der allgemeinen Formel IV

B-A-(B-A-)ₜ-B (D²)ᵤ IV

in der D² eine der folgenden enantiomeren Gruppen ist:
eine lineare C₁- bis C₂₀-Alkylgruppe, die einmal durch die Gruppierungen -COO- und -OCO- oder bis zu zweimal durch -O- unterbrochen sein kann. Eine lineare C₁- bis C₂₀-Alkylgruppe, die an ein oder zwei Kohlenstoffatomen durch Fluor, Chlor, Brom, Cyano, Trifluormethyl oder Methyl asymmetrisch substituiert ist, und die einmal durch die Gruppierung -CO-O- oder bis zu zweimal durch die Gruppierung -O- unterbrochen sein kann, oder
eine lineare C₃- bis C₆-Alkylgruppe, die durch die Gruppierung 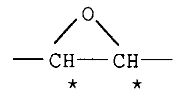
unterbrochen ist,
mit der Maßgabe, daß diese enantiomeren Gruppen D² durch -O-, -CO-O- oder -O-CO- mit A verknüpft sein können.

Beispiele gut geeigneter Gruppen D² sind:

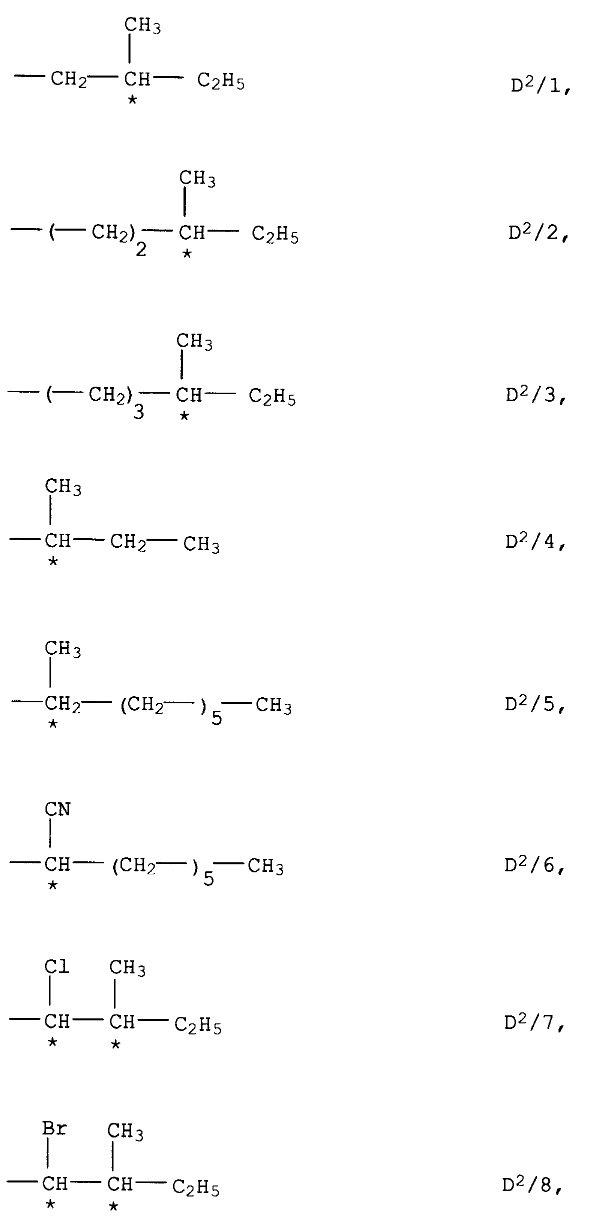

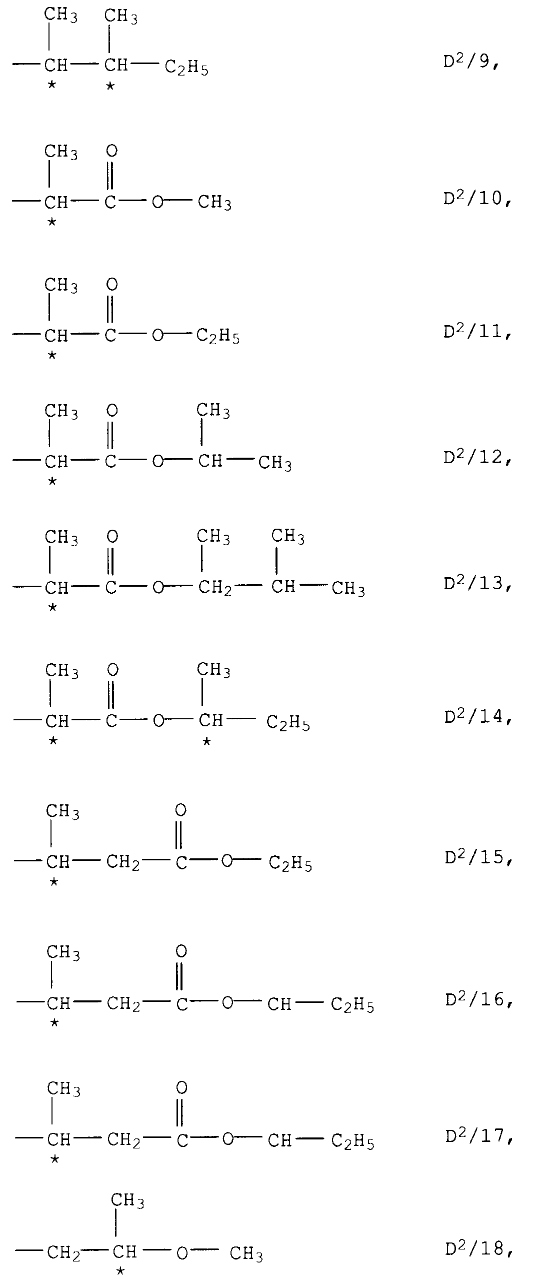

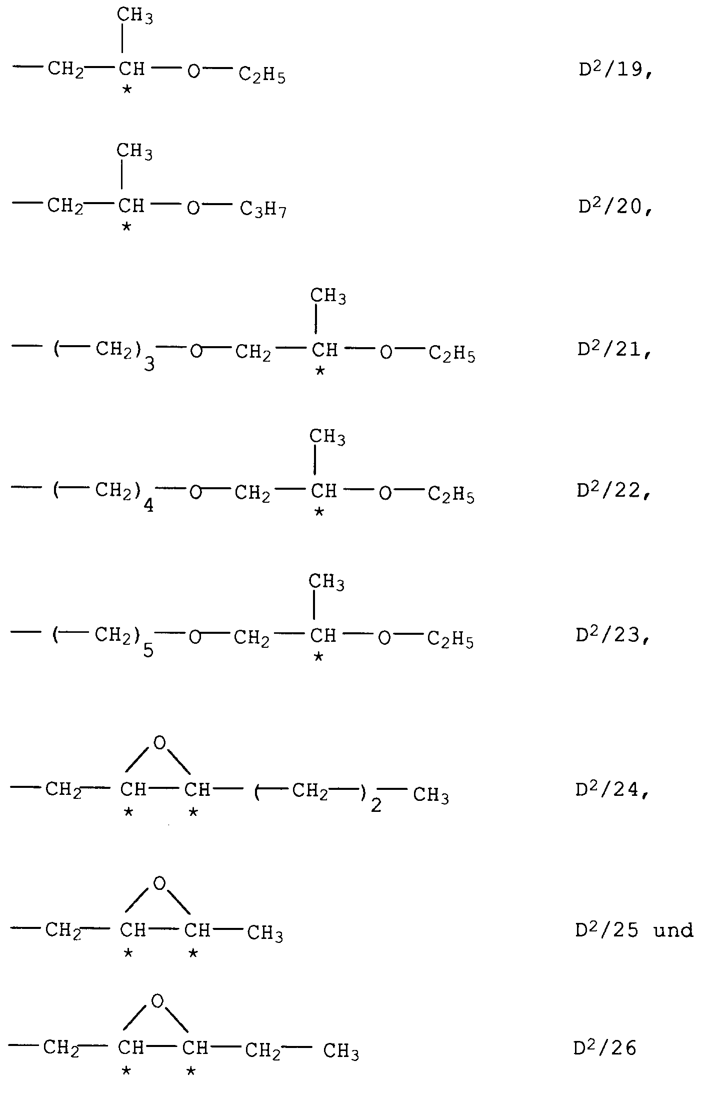

―(CH₂)ᵥ―H, D²/27

mit v = 4 bis 15
von denen die Gruppen ²/6, D²/7, D²/11, D²/15, D²/17 bis D²/21, D²/24 und D²/27 besonders gut geeignet sind.

Eine bevorzugte mesogene Gruppe ist: 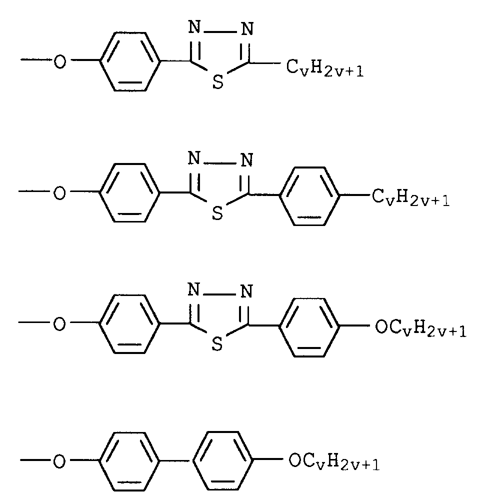
Für die Herstellung der flüssigkristallinen Verbindungen I gilt allgemein das Prinzip, Alkohole Z¹(H)ₘ mit Carbonsäurehalogeniden M-Y-R¹-CO-Hal, wobei Hal für Chlor oder Brom steht, oder Carbonsäuren Z(H)ₘ oder deren Derivate wie Säurechloride Z¹(Cl)ₘ oder Anhydride (Z¹)₂O mit Alkoholen M-Y-R¹-OH umzusetzen.

Im einzelnen sind die flüssigkristallinen Verbindungen I nach folgenden Methoden erhältlich:
Man geht von einer Carbonsäure M-Y-R¹-COOh aus und setzt diese in bekannter Weise mit Thionylchlorid in Gegenwart von Dimethylformamid zum Carbonsäurechlorid M-Y-R¹-CO-Cl um. Im nachfolgenden Schritt ist durch Umsetzung des Säurechlorids M-Y-R¹-CO-Cl mit einem Alkohol Z¹(H)ₘ in Gegenwart von Pyridin die Verbindung I erhältlich.

Die Alkoholyse von Carbonsäurechloriden ist in zahlreichen Ausgestaltungen allgemein bekannt, so daß sich nähere Ausführungen hierzu erübrigen. Es ist lediglich hinzuzufügen, daß es sich meistens empfiehlt, das Säurechlorid im Überschuß einzusetzen, um den Alkohol quantitativ umzusetzen. Nach der Isolierung des Produktes I kann eine zusätzliche Reinigung mittels Gelchromatographie und anschließende Umkristallisation vorgenommen werden, wozu sich meistens Methylenchlorid-Methanol-Gemische eignen.

Eine weitere Möglichkeit zur Herstellung der flüssigkristallinen Verbindung I besteht in der sauer katalysierten Veresterung der Carbonsäuren U¹(H)ₘ, der Säurechloride Z¹(Cl)ₘ und der Anhydride (Z¹)₂O mit Alkoholen M-Y-R¹-OH.

Die Veresterung ist in zahlreichen Ausgestaltungen hinsichtlich der Säure, der Temperatur und der Reaktionszeit allgemein bekannt.

Eine Variante zur Umsetzung von Z¹(H)ₘ mit Carbonsäurechloriden stellt die Umsetzung von Z¹(H)ₘ mit ω-Bromcarbonsäurechloriden Br-R¹-CO-Cl und die anschließende Veretherung des Zwischenproduktes Z¹-CO-R¹-Br mit einer alkoholischen Verbindung HO-M zur Verbindung I dar.

Für die Herstellung der flüssigkristallinen Verbindungen II gilt allgemein das Prinzip, phenolische Verbindungen Z²(OH)ₙ mit Verbindungen, die sich von Verbindungen des Typs M-Y-R¹-X²-H ableiten oder deren Derivate sind, oder mit einem Alkylbromid M-Y-R¹ umzusetzen.

Ferner erhält man die Verbindungen II in an sich bekannter Weise durch Umsetzung der Verbindung Z²(OH)ₙ mit ω-Bromcarbonsäurechlorid Br-R¹-CO-Cl über die Stufe eines Zwischenproduktes Z²(-O-CO-R¹-Br)ₙ, welches anschließend mit der Verbindung HO-M verethert wird.

Eine weitere Möglichkeit zur Herstellung der Verbindungen II stellt die Veresterung von Carbonsäuren Z²(COOH)ₙ, Säurechloriden Z²(COCl)ₙ und Anhydriden (Z²CO)₂ mit Verbindungen des Typs M-Y-R¹-OH dar, die auf übliche Weise durchgeführt wird.

Die erfindungsgemäßen flüssigkristallinen Verbindungen I und II sind in hoher Reinheit mit einem einheitlichen Molekulargewicht herstellbar und zeigen eine hohe Fließviskosität.

Die erfindungsgemäßen flüssigkristallinen Verbindungen I und II eignen sich hervorragend allein, in Mischungen untereinander und mit anderen flüssigkristallinen und/oder nicht flüssigkristallinen Verbindungen in Form fester oder flüssigkristalliner optisch anisotroper Medien für die Darstellung und Speicherung von Informationen. Darüber hinaus eignen sie sich zur Herstellung anisotroper fester optischer Medien für optische Bauteile wie Kompensatoren, Polarisatoren, Prismen, Platten mit optischer Drehung und elektrooptischen Speicherdisplays.

### Beispiele

Die Phasenübergänge wurden im Polarisationsmikroskop (Leitz, Ortholux II Pol BK, Mettler-Heiztisch FP 82 HT, Mettler-Steuergerät FP 80), Heizrate 10°C/min, und durch DSC-Messungen (Perkin-Elmer, DSC-7), Heizrate 20°C/min, bestimmt.

Die folgende Symbolik kennzeichnet die Phasen. Ein Phasenwechsel findet bei den gemessenen Phasenumwandlungstemperaturen statt.
- k: = kristallin
- g: = Glasstufe
- n: = nematisch
- ch: = cholesterisch
- s: = smektisch
- i: = isotrop
- S_{A}: smektisch A
- S_{C}: smektisch C

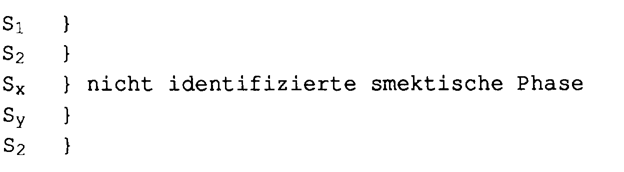

Die Umwandlungstemperaturen (°C) der erfindungsgemäßen Verbindungen sind jeweils bei den Beispielen angegeben. Die Temperaturen in Klammern bedeuten monotrope Umwandlungen der Phasen. Darunter sind Phasenübergänge zu verstehen, die nur in einer Richtung ablaufen, z.B. dann, wenn der flüssigkristalline Zustand nur durch Unterkühlung der Schmelze erreichbar ist. Der Schmelzpunkt liegt dann höher als der Klärpunkt.

### Herstellung der flüssigkristallinen Verbindungen

### Beispiel 1

### Herstellung von Tri-(ω-Brombutanoyl-)-glyceriden

5 mmol Glycerol, 16,5 mmol ω-Bromcarbonsäure und 1,65 mmol DMAP (Dimethylaminopyridin) werden in 25 ml absolutem Chloroform gelöst in einem 100 ml Einhalskolben mit Magnetrührer und Septum vorgelegt und mit Eisbadkühlung auf 5°C abgekühlt. Danach werden 16,5 mmol DCC (Dicyclohexylcarbodiimid) in 25 ml absolutem Chloroform gelöst, langsam mit einer Spritze zugetropft. Man läßt auf Raumtemperatur erwärmen und ca. 72 h rühren. Anschließend wird der weiße Niederschlag abgesaugt (über eine 2 bis 3 cm starke Kieselgurschicht) und mit Chloroform gewaschen. Das Filtrat wird am Rotationsverdampfer eingeengt und der Rückstand chromatografisch gereinigt.
Laufmittel: Chloroform:Methanol/10:0,04
Ausbeute: 96 %.

### Beispiel 2

Herstellung von Glycerol-tri-(4-(2-heptyl-1,3,4-thiadiazol-5-yl)-phenol)-butylcarboxylat,

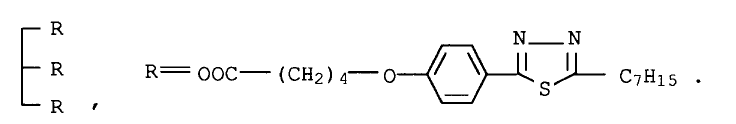

In einem 100 ml Zweihalskolben mit Rückflußkühler und Magnetrührer werden 0,5 mmol Tri-(ω-brombutanoyl)-glycerid, 1,8 mmol 4-(2-n-heptyl-1,3,4-thiadiazol-5-yl)-phenol, 5 mmol frisch geglühtes K₂CO₃ und 0,5 mmol KJ in 20 ml absolutem Diethylketon oder Butanon gelöst und unter Rückfluß und Schutzgas 40 bis 60 h gerührt. Nach Beendigung der Reaktion wird das Lösungsmittel unter Vakuum abdestilliert, der Rückstand in Chloroform aufgenommen und vorsichtig mit 10%iger HCL angesäuert, bis eine klare Lösung entsteht. Die organische Phase wird mehrmals mit gesättigter NaHCO₃- und anschließend mit gesättigter NaCl-Lösung neutral gewaschen, über NaSO₄ getrocknet und das Lösungsmittel abgezogen.

Der Rückstand wird mehrmals aus absolutem Ethanol umkristallisiert.
Ausbeute: 10 %
Phasenverhalten: K 81 S_{c} 98 I

### Beispiel 3

Analog Beispiel 2 wurde Glycerol-tri-(4-(2-nonyl-1,3,4-thiadiazol-5-yl)-phenol)-butylcarboxylat, 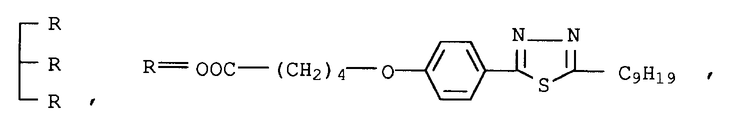
hergestellt.
Ausbeute: 5 %
Phasenverhalten: K 88 S_{c} 113 I

### Beispiel 4

Analog Beispiel 2 wurde Glycerol-tri-(4-(2-pentadecyl-1,3,4-thiadiazol-5-yl)-phenol)-butylcarboxylat, 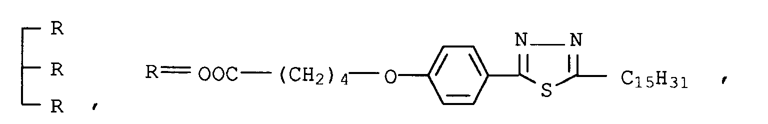
hergestellt.
Ausbeute: 16,5 %
Phasenverhalten: K ? S_{c} 130 I

### Beispiel 5

Analog Beispiel 2 wurde Glycerol-tri-(4-(2-p-octyloxyphenyl-1,3,4-thiadiazol-5-yl)-phenol)-butylcarboxylat, 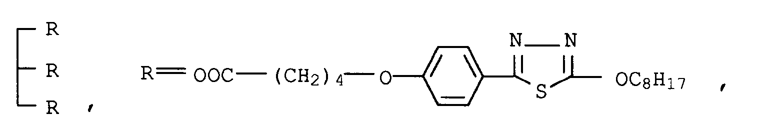
hergestellt.
Ausbeute:
Phasenverhalten: K 143 S_{c} 241 I

### Beispiel 6

Analog Beispiel 1 wurde Tri-(ω-bromessigsäure)-glycerid hergestellt.
Ausbeute: 94 %.

### Beispiel 7

Analog Beispiel 2 wurde Glycerol-tri-(4-(2-nonyl-1,3,4-thiadiazol-5-yl)-phenol)-methylcarboxylat, 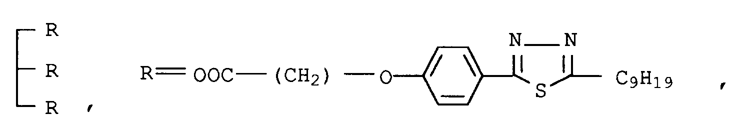
Ausbeute: 5 %
Phasenverhalten: K 260 Sₓ 270 S_{y} 300 Z.

### Beispiel 8

Analog Beispiel wurde Tri-(ω-bromdecanoyl)-glycerid hergestellt.
Ausbeute: 82 %

### Beispiel 9

Analog Beispiel wurde Glycerol-tri-(4-(2-nonyl-1,3,4-thiadiazol-5-yl)-phenol)-decylcarboxylat, 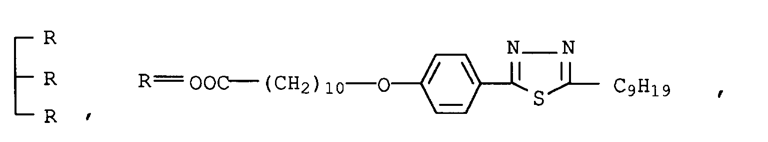
hergestellt.
Ausbeute: 50 %
Phasenverhalten: K ? S_{z} 103 I.

### Beispiel 10

Analog Beispiel 9 wurde Glycerol-tri-(4-(2-heptyl-1,3,4-thiadiazol-5-yl)-phenol)-decylcarboxylat, 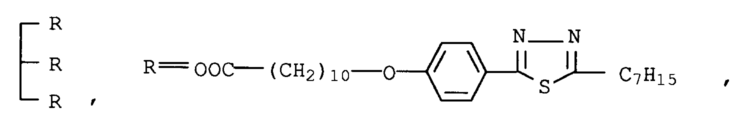
hergestellt.
Ausbeute: 27,6 %
Phasenverhalten: K ? S_{z} 102 I

### Beispiel 11

Analog Beispiel 1 wurde tetra-(ω-brombutanoyl)-pentaerythrit hergestellt.
Ausbeute: 51 %.

### Beispiel 12

Analog Beispiel 2 wurde Pentaerythrit-tetra-(4-(2-nonyl-1,3,4-thiadiazol-5-yl)-phenol)-butylcarboxylat, 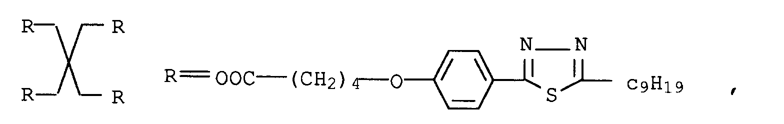
hergestellt.
Ausbeute: 37,2 %
Phasenverhalten: K 121 (S_{c} 118) I

### Beispiel 13

Analog Beispiel 1 wurde Hexa-(ω-brombutanoyl)-mannit hergestellt.
Ausbeute: 46,1 %.

### Beispiel 14

Analog Beispiel 2 wurde Mannit-hexa-(4-(2-nonyl-1,3,4-thiadiazol-5-yl)-phenol)-butylcarboxylat,

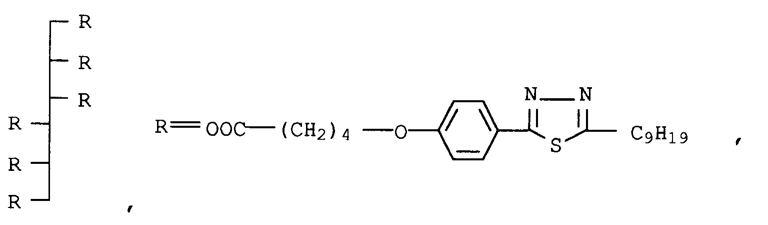

### Beispiel 15

### Herstellung von 4-Dodecyloxy-4'-ethylbutoxylat-biphenyl

In einem 250 ml Dreihalskolben mit Rückflußkühler und Magnetrührer werden 30 mmol dodecyloxybiphenylol, 35 mmol Brombuttersäureethylester, 30 mmol frisch geglühtes Kaliumcarbonat (K₂CO₃) und 9 mmol Kaliumjodid (KJ) in 50 ml absolutem Diethylketon gelöst und 60 bis 72 h bei Raumtemperatur gerührt. Nach Beendigung der Reaktion wird das Lösungsmittel abgezogen und der Rückstand in Chloroform aufgenommen, mit 10%iger HCl angesäuert und mit gesättigter NaHCO₃-Lösung neutral gewaschen. Nach zweimaligem Umkristallisieren aus absolutem Ethanol wird der Rückstand mit Chloroform:Ethanol/10:0,08 chromatografiert.
Ausbeute: 84,7 %
Schmelzpunkt: 99°C.

### Beispiel 16

### Herstellung von 4-Butoxycarbonsäure-4'-dodecyloxy-biphenyl

In einem 500 ml Einhalskolben mit Rückflußkühler und Magnetrüher werden 19 mmol der Verbindung aus Beispiel 15, 66,7 mmol KOH, 100 ml Ethanol und 50 ml Wasser vorgelegt und 6 h unter Rückfluß erhitzt. Nach dem Abkühlen wird das Reaktionsgemisch vorsichtig in konzentrierter HCl-Lösung angesäuert und anschließend mit Waser neutral gewaschen. Das Lösungsmittel wird abgezogen und der Rückstand mehrmals aus Eisessig umkristallisiert. Danach wird der Rückstand nochmals mit Methanol und anschließend mit Ether aufgekocht und abgesaugt.
Ausbeute: 90,5 %.

### Beispiel 17

Herstellung von Glycerol-tri-(4-dodecyloxy-biphenylol-propylcarboxylat, 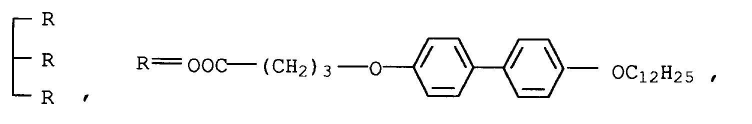
0,8 mmol Glycerol, 2,8 mmol der Verbindung aus Beispiel 16 und 0,28 mmol DMAP (Dimethylaminopyridin) werden in 25 ml absolutem Chloroform in einem 100 Einhalskolben mit Magnetrührer und Septum suspendiert. Dazu tropft man unter Eiskühlung 5,76 mmol DCC (Dicyclohexylcarbodiimid) in 10 ml absolutem Chloroform gelöst. Anschließend läßt man bei Raumtemperatur 72 h rühren. Danach wird der weiße Niederschlag abgesaugt, das Filtrat eingeengt und der Rückstand chromatografiert (Chloroform:Eisessig/10:0,02). Anschließend wird nochmals aus Essigester umkristallisiert.
Ausbeute: 6,8 %
Phasenverhalten: K 144 S_{A} 162 I

### Beispiel 18

Analog Beispiel 17 wurde Pentaerythrit-tetra-(4dodecyloxy-biphenylol-propylcarboxylat), 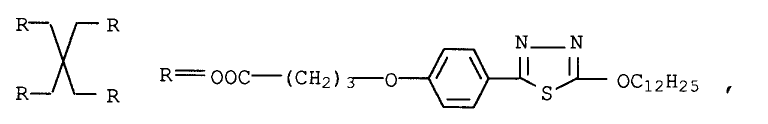
hergestellt.
Ausbeute: 5,5 %
Phasenverhalten: K ? S₁ 143 S₂ 170 S_{A} 173 I.

## Patentansprüche

1. Definierte, oligomere flüssigkristalline Verbindung mit smektisch flüssigkristallinen Phasen der allgemeinen Formeln I und II,
Z¹-(X¹-R¹-Y-M)ₘ I
Z²-(X²-R¹-Y-M)ₙ II.
in denen die Variablen folgende Bedeutung haben:
Z¹ der Alkoholat- oder Säurerest Z¹/1 eines m-wertigen aliphatischen Alkohols bzw. einer m-wertigen aliphatischen Carbonsäure mit 3 bis 30 C-Atomen;
der Alkoholat- oder Säurerest Z¹/2 eines m-wertigen cycloaliphatischen Alkohols bzw. einer m-wertigen cycloaliphatischen Carbonsäure mit 5- oder 6-Ringgliedern;
für den Fall m = 3 der stickstoffhaltige Rest Z¹/3, 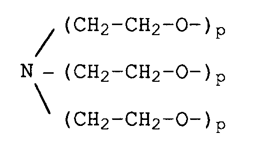 wobei p 1 oder 2 sein kann;
für den Fall M = 3 ein Rest der Strukturen Z¹/4a-d 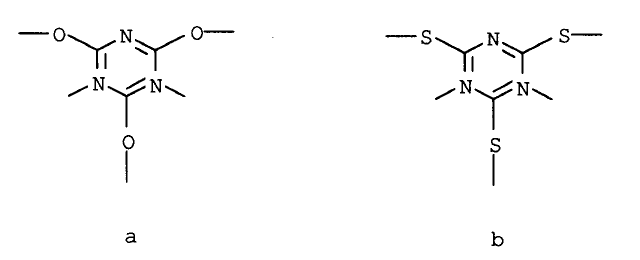 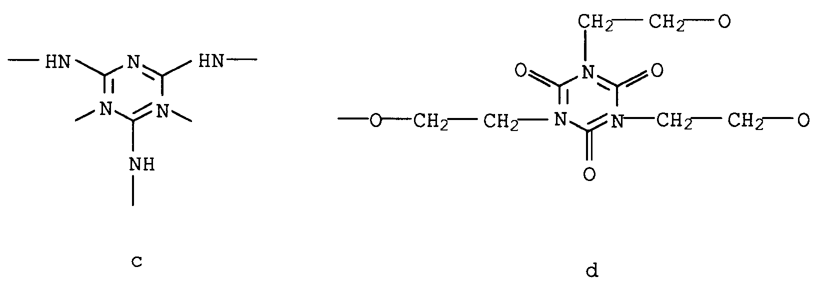 für den Fall m = 3 oder m = 4 der Säurerest Z¹/5 der Nitrilotriessigsäure bzw. der Ethylendiaminotetraessigsäure;
der Rest Z¹/6 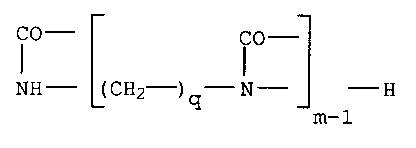 wobei q 2 oder 3 sein kann;
Z² der n-wertige Rest Z²/1 eines Benzols, 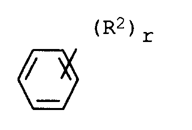 wobei R² Halogen, Cyano, Nitro, C₁- bis C₁₀-Alkyl, C₁- bis C₁₀-Alkoxy, C₁- bis C₁₀-Alkoxycarbonyl, C₁- bis C₁₀-Acyloxy oder Reste, die in vicinaler Stellung zu einem Ring verbunden sind, bedeuten kann; r für Null bis 3 steht und die Reste R² im Falle r > 1 gleich oder verschieden sein können;
der mehrkernige Rest Z²/2, 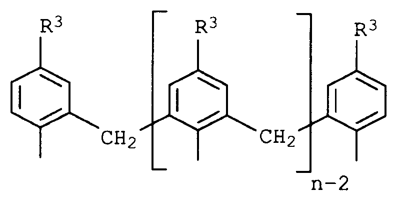
wobei R³ C₁- bis C₄-Alkyl, C₁- bis C₄-Alkoxy oder Halogen bedeuten kann;
für den Fall n = 3 der phosphorhaltige Rest Z²/3, 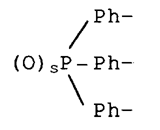 wobei s Null oder 1 sein kann;
X¹ eine chemische Bindung oder eine -CO- Gruppe;
X² Sauerstoff, Schwefel, -CO-O-, -O-CO-, -SO₂-, -SO₂-O, -O-SO₂-O, -NR⁴-, -CO-NR⁴-, -NR⁴-O- oder 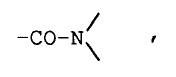 wobei R⁴ Wasserstoff oder C₁- bis C₈-Alkyl bedeuten kann, und die Maßgabe gilt, daß X² im Falle des mehrkernigen Restes Z²/2 nur -O- oder -O-CO- bedeutet;
m 3 bis 6, mit der Maßgabe, daß m gleich oder kleiner der Anzahl der C-Atome in Z¹ ist;
n 3 bis 6;
R¹ eine C₂- bis C₂₀-Brücke mit 2 bis 12 Brückengliedern, die durch Sauerstoff, Schwefel oder -NR⁴- unterbrochen sein können, und wobei diese Heteroeinheiten jeweils durch mindestens 2 C-Atome getrennt sind;
Y eine chemische Bindung, Sauerstoff, Schwefel, -CO-O-, -O-CO-, -NR⁴-, -CO-NR⁴- oder -NR⁴-CO-;
M eine mesogene Gruppe, die sich von einer Verbindung ableitet, welche in der flüssigkristallinen Phase eine Anisotropie der Dielektrizitätskonstanten ε mit |Δε| > 0,3 aufweist und/oder welche optisch aktiv ist.

2. Flüssigkristalline Verbindungen nach Anspruch 1, in denen die mesogene Gruppe M eine Gruppe der allgemeinen Formel III ist,
-A-(B-A-)ₜ-B-(D¹)ᵤ III
in der die Variablen die folgende Bedeutung haben:
A eine 1,4-Phenylen- oder 2,6-Naphthylengruppe, die bis zu zwei Stickstoffatome als Heteroatome enthalten kann, und die bis zu zwei der folgenden Substituenten tragen kann: Fluor, Chlor, Brom, Nitro oder Cyano;
eine 1,3,4-Thiadiazolylgruppe;
eine 1,4-Cyclohexylengruppe, die bis zu zwei der folgenden Heteroatome in jeweils nicht benachbarter Stellung enthalten kann: Sauerstoff, Schwefel oder Stickstoff;
B eine chemische Bindung oder eines der folgenden Brückenglieder:
-CO-O-, -O-CO-, -CH₂-CH₂-, -CH₂-O-, -O-CH₂-, -CH₂-S-, -S-CH₂-, -C≡C-, -CH=CH-, -CH=N-, -N=CH-, -N=N-, 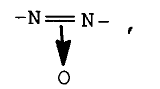 -CH=CH-CO-O- oder -O-CO-CH=CH-,
D¹ einer der folgenden Substituenten:
Fluor, Chlor, Brom, Nitro, Cyano, -OCFH₂, -OCF₂H, -OCF₃, -O-CO-R⁵ oder -CO-O-R⁵,
wobei R⁵ ein linearer C₁- bis C₂₀-Alkylrest, der durch Sauerstoff unterbrochen sein kann, und der durch Fluor, Chlor, Brom, Cyano oder Methyl asymmetrisch substituiert sein kann, bedeutet;
t 1 bis 4, mit der Maßgabe, daß die Gruppen A und B verschieden sein können,
u 1, wenn D¹ mit einem aromatischen Ring verknüpft, und 1 oder 2, wenn D¹ mit einem Cyclohexylenring verknüpft ist.

3. Flüssigkristalline Verbindungen nach Anspruch 1, in denen die mesogene Gruppe M eine Gruppe der allgemeinen Formel IV ist,
B-A-(B-A-)ₜ-B (D²)ᵤ IV
in der D² eine der folgenden enantiomeren Gruppen ist:
eine lineare C₁- bis C₂₀-Alkylgruppe, die an ein oder zwei Kohlenstoffatomen durch Fluor, Chlor, Brom, Cyano, Trifluormethyl oder Methyl asymmetrisch substituiert ist, und die einmal durch die Gruppierung -CO-O- oder bis zu zweimal durch die Gruppierung -O- unterbrochen sein kann, oder
eine lineare C₃- bis C₆-Alkylgruppe, die durch die Gruppierung 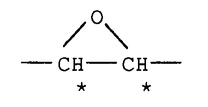 unterbrochen ist,
mit der Maßgabe, daß diese enantiomeren Gruppen D² durch -O-, -CO-O- oder -O-CO- mit A verknüpft sein können.

4. Verfahren zur Herstellung der flüssigkristallinen Verbindungen I gemäß den Ansprüchen 1 bis 3, dadurch gekennzeichnet, daß man die Verbindungen Z¹(H)ₘ, Z¹(Cl)ₘ oder (Z¹)₂O in an sich bekannter Weise mit einem Carbonsäurehalogenid M-Y-R¹-CO-Hal bzw. einem Alkohol M-Y-R¹-O-H umsetzt, wobei Hal für Chlor oder Brom steht.

5. Verfahren zur Herstellung der flüssigkristallinen Verbindungen I gemäß den Ansprüchen 1 bis 3, dadurch gekennzeichnet, daß man die Verbindung Z¹(H)ₘ in an sich bekannter Weise mit einem ω-Bromcarbonsäurechlorid Br-R¹-CO-Cl umsetzt und das Zwischenprodukt Z¹-CO-R¹-Br mit der Verbindung HO-M verethert.

6. Verfahren zur Herstellung der flüssigkristallinen Verbindungen II gemäß den Ansprüchen 1 bis 3, dadurch gekennzeichnet, daß man die Verbindung Z²(OH)ₙ in an sich bekannter Weise mit Verbindungen, die sich von der Verbindung M-Y-R¹-X²-H ableiten, bzw. einem Alkylbromid M-Y-R¹-Br umsetzt.

7. Verfahren zur Herstellung der flüssigkristallinen Verbindungen II gemäß den Ansprüchen 1 bis 3, dadurch gekennzeichnet, daß man die Verbindung Z²(OH)ₙ in an sich bekannter Weise mit ω-Bromcarbonsäurechlorid Br-R¹-CO-Cl umsetzt und das Zwischenprodukt Z²-O-CO-R¹-Br mit der Verbindung HO-M verethert.

8. Verfahren zur Herstellung der flüssigkristallinen Verbindungen II gemäß den Ansprüchen 1 bis 3, dadurch gekennzeichnet, daß man die Verbindungen Z²(COOH)ₙ, Z²(COCl)ₙ und (Z²-CO)₂O in an sich bekannter Weise mit einer Verbindung M-Y-R¹-OH umsetzt.

9. Verwendung der flüssigkristallinen Verbindungen I und II gemäß den Ansprüchen 1 bis 3 allein, in Mischungen untereinander und mit anderen flüssigkristallinen und/oder nicht flüssigkristallinen Verbindungen in Form fester oder flüssigkristalliner optisch anisotroper Medien für die Darstellung und Speicherung von Informationen.
